# FASCICULE DE BREVET EUROPEEN

(11) **EP 0 831 865 B1**
(45) Date de publication et mention de la délivrance du brevet: **17.04.2002**
(21) Numéro de dépôt: 96920900.6
(22) Date de dépôt: 05.06.1996
(51) Int. Cl.: A61K 35/78, A61K 7/06, A61P 17/00, A61P 17/04, A61P 17/08

(54) **COMPOSITION CAPILLAIRE COMPRENANT UN EXTRAIT DE MYRTE, SON PROCEDE DE PREPARATION ET SON UTILISATION NOTAMMENT POUR UN TRAITEMENT ANTIPELLICULAIRE**
MYRTEEXTRAKT ENTHALTENDES HAARPFLEGEMITTEL, VERFAHREN ZUR HERSTELLUNG UND VERWENDUNG INSBESONDERE FÜR EINE ANTISCHUPPENBEHANDLUNG
HAIR COMPOSITION COMPRISING A MYRTLE EXTRACT, PREPARATION PROCESS AND UTILIZATION PARTICULARLY FOR AN ANTI-DANDRUFF TREATMENT

(30) Priorité: 07.06.1995 FR 9506695
(43) Date de publication de la demande: 01.04.1998
(73) Titulaire: Pierre Fabre Dermo-Cosmetique, 92100 Boulogne (FR)
(72) Inventeur: MAMATAS, Stylianos Résidence les Cyclades, F-31400 Toulouse (FR); JEANJEAN, Michel, F-31320 Castanet-Tolosan (FR); LAGARDE, Isabelle, F-31540 Maurens (FR); TEYSSEYRE, Valérie, F-31520 Ramonville-Saint-Agne (FR); FABRE, Bernard, F-31450 Belberaud (FR)
(74) Mandataire: Ahner, Francis
(86) Numéro de dépôt international: FR9600843
(87) Numéro de publication internationale: WO9640180

(56) Documents cités:
- EP-A- 0 347 493
- DATABASE WPI Section Ch, Week 9223 Derwent Publications Ltd., London, GB; Class D21, AN 92-190090 XP002013066 & JP,A,04 128 214 ( FUKUI Y) , 28 Avril 1992

## Description

L'invention a pour objet une composition capillaire comprenant un extrait de myrte.

Elle vise également son procédé de préparation et son utilisation notamment pour un traitement antipelliculaire.

On connaît les désagréments causés par certaines affections capillaires, telles que les pellicules, les séborrhées ou encore les manifestations cutanées.

Outre la gêne qu'ils occasionnent du point de vue esthétique, ils peuvent être à l'origine de manifestations inflammatoires voire infectieuses.

Dans certains cas, des complications peuvent même intervenir, par exemple lorsque ces affections se manifestent chez des sujets présentant d'autres troubles cutanés comme de l'eczéma.

La recherche de compositions appropriées au traitement de ces diverses affections a conduit à la formulation de nombreux produits.

Cependant, la plupart du temps, ils mettent en jeu des matières premières de synthèse, c'est-à-dire des matières premières souvent coûteuses et longues à préparer.

Qui plus est, ces matières de synthèse suscitent parfois des allergies et leur application peut donc avoir pour effet, en réalité, d'aggraver les affections traitées.

Il serait donc souhaitable de fournir une composition propre à traiter les affections mentionnées ci-dessus en ayant recours à des matières premières naturelles. En effet, si celles-ci ne suppriment pas totalement les risques d'allergies, en tout cas elles les réduisent souvent.

En fait, dans le domaine cosmétique ou médical, on sait déjà mettre en oeuvre, pour beaucoup de types de soins, des produits d'origine naturelle.

Par exemple, le brevet FR 2 504 551 décrit des compositions aromatiques possédant certaines propriétés biologiques et les médicaments dermatologiques les renfermant. Ces compositions sont notamment destinées au traitement d'affections et de traumatismes des tissus cutanés. Ce document indique une liste de plantes qui pourraient être utilisées dans ces compositions. Par exemple, sont citées les labiées, les géraniacées, les rutacées et les myrtacées.

De même, le brevet FR 2 669 032 enseigne un procédé particulier de fabrication d'une préparation à haute teneur en d-alpha-tocophérol. Ce procédé fait appel à des feuilles de plantes choisies parmi les familles Caprifoliaceae, Lauraceae, Myrtaceae, Ericaceae. La préparation ainsi obtenue est utilisée pour ses propriétés anti-oxydantes.

On peut citer encore le document FR 2 296 401 qui décrit des compositions capillaires à base de progestérone et de placenta. Ces compositions comprennent en outre, en tant que produits secondaires, des extraits de myrte et d'oignon, d'hamamelis et de millepertuis, ou des extraits d'algues. On note que ces produits secondaires sont présentés de façon indéfinie, notamment, le rôle de chacun n'est nullement établi.

On peut citer enfin le brevet DE 3 434 496 qui décrit des compositions destinées à activer la croissance des cheveux. Ces compositions sont à base de pommes acides, de citrons, d'huile d'olive, d'oeufs et de myrtol. Là encore, le rôle de chaque constituant n'est nullement établi. Tout au plus, peut-on tirer de ce document, qu'il faut mettre en oeuvre tous les constituants cités pour obtenir l'effet désiré.

Aucun de ces documents illustrant l'état de la technique antérieure n'indique clairement le rôle de chaque substance naturelle dans le traitement considéré. Ils ne font même pas véritablement la distinction entre les matières utilisées comme principes actifs et celles servant en fait d'excipients.

Or, la Demanderesse a trouvé, à l'issue de recherches longues et approfondies, qu'un extrait naturel particulier, à savoir l'extrait de myrte, était efficace à l'égard de certaines affections capillaires déterminées, à savoir principalement les pellicules, les séborrhées, et les affections prurigineuses.

En d'autres termes, la Demanderesse a mis en évidence, contre toute attente, que l'extrait de myrte constitue un principe actif efficace à l'égard des affections précitées.

Par conséquent, le mérite de la Demanderesse est d'autant plus grand qu'elle a non seulement opéré une sélection parmi les nombreuses plantes utilisées pour des compositions traitantes, mais elle a, en outre, précisément déterminé les applications capillaires de cet extrait de myrte.

Le myrte était célébré en Perse ancienne et en Egypte, chez les Hébreux qui le faisaient intervenir dans les rites religieux. La présence du myrte dans la littérature grecque ancienne est importante, ce qui laisse supposer un emploi étendu des fruits (on préparait des vins aromatisés avec ces baies, utilisées également directement comme condiments). Mais de myrtos, nom grec à l'étymologie inconnue, ont dérivés le latin myrtus pour notre plante et plusieurs noms arméniens et iraniens. Toutefois, ce myrtus latin a servi aussi à désigner le fragon ou petit-houx - également arbrisseau à feuilles persistantes. Et le nom de myrtille rappelle une autre confusion des Anciens. Le myrte servait à la confection de couronnes pour les ovations ou petits triomphes et cette réputation flatteuse réapparaîtra en France à la Renaissance - lisons Ronsard. Dioscoride et Pline recommandaient la plante pour de nombreux usages. Plus tard, Ambroise Paré fera bon usage du myrte pour traiter les plaies. Le jus des baies passait pour protéger les estomacs fragiles et accroître la diurèse. La décoction des graines servait en cosmétique pour le noircissement des cheveux et réduisait l'inflammation oculaire. Au XVII^{e} siècle, l'"eau d'ange", obtenue par distillation d'eau sur des fleurs, connaissait un vif succès. Au XIX^{e} siècle, on préconisait encore des infusions de feuilles et de fleurs contre leucorrhées, affections pulmonaires putrides et hémorroïdes. Mais si les Anciens avaient plus ou moins confusément pris conscience des propriétés antibactériennes de l'huile essentielle du myrte, sans soupçonner évidemment l'existence des germes, la plante allait vers l'oubli lorsque, en 1878, Linarix relança son intérêt en pathologie respiratoire : elle se présentait comme stomachique, stimulante, astringente, mais plus encore antiseptique, désinfectante et parasiticide - propriété mieux appréciée de nos jours. La forte teneur en tanins des feuilles et tiges et surtout des galles de tiges les a fait utiliser en Italie pour le tannage des peaux.

Le myrte est un arbuste de deux à trois mètres de haut à tige roussâtre, feuilles opposées deux à deux, rarement disposées par trois, coriaces, persistantes, à court pétiole et limbe ovalaire acuminé. Il présente aussi des fleurs blanches disposées une par une à l'aisselle des feuilles, odorantes, à nombreuses étamines à longs filets. Il donne naissance à un fruit ovoïde, de la grosseur d'un pois, charnu, vert, puis bleuâtre à maturité de saveur "résineuse" et odeur aromatique. Toutes les parties de la plante contiennent des poches shizogènes à huile essentielle, visibles par observation des feuilles en transparence et responsables d'une odeur forte après contusion. Le myrte se rencontre spontanément dans les bois et coteaux des pays du pourtour méditerranéen et en proche Asie méridionale. Il est une composante des maquis (à sol acide) et garrigues (à sol alcalin). On connaît deux variétés bactica et italica.

En fait, la qualité des compositions traitantes capillaires est effectuée en fonction de différents critères. Pour évaluer l'action antipelliculaire des compositions, on contrôle leur activité fongicide, leur activité anti-inflammatoire et leur activité antiradicalaire.

De même, pour évaluer leur activité antiséborrhéique, on contrôle la prolifération du sébum sous l'action des compositions, c'est-à-dire l'inhibition du Pityrosporum.

Et pour cause, l'épiderme du cuir chevelu est constitué de plusieurs couches de cellules. On distingue en allant de l'intérieur vers l'extérieur, la couche basale germinative, siège des mitoses, la couche épineuse formée de plusieurs rangées de cellules qui tendent à s'aplatir vers le haut, la couche granuleuse dont les cellules aplaties ont un noyau qui commence à dégénérer, et enfin, la couche superficielle constituée de cellules kératinisées sans noyau, qui se détachent en petites lamelles.

On considère, en général, que si les amas de cellules qui se détachent sont invisibles à l'oeil nu, c'est qu'ils sont de taille inférieure à deux cents microns. Par contre, s'ils dépassent une taille de deux cents microns, ils sont visibles et peuvent constituer un état pelliculaire.

Dans ce cas, on observe en plus du phénomène d'agrégation, une augmentation de la vitesse de migration des cellules vers l'extérieur, la vitesse normale est de vingt et un jours, ainsi qu'une augmentation de l'index mitotique, donc une prolifération cellulaire.

On peut distinguer cliniquement deux états pelliculaires, à savoir les pellicules grasses ou pityriasis stéatoïde et les pellicules sèches ou pityriasis simplex.

Les pellicules grasses sont des squames de grandes tailles, plus épaisses et plus grasses que dans le cas du simplex. Elles peuvent former un enduit gras et desquamatif à la surface du cuir chevelu induisant une inflammation et un prurit séborrhéique important. Ce pityriasis stéatoïde est souvent rattaché à une dermite séborrhéique.

Les pellicules sèches sont des squames de petites tailles, se détachant facilement de l'épiderme qui, à l'examen clinique, n'apparaît pas inflammatoire. Ce pityriasis simplex est souvent accompagné de prurit sans érythème.

Si on ne fait pas de distinction majeure entre pityriasis stéaroïde et simplex, les pellicules sont alors considérées comme une réponse à un état inflammatoire qui n'est pas toujours détectable cliniquement et qui peut être accompagné de séborrhée, le pityriasis stéaroïde est jugé comme un état grave du pityriasis simplex.

L'étiologie des pellicules n'est pas totalement connue et est vraisemblablement due à plusieurs facteurs.

Le facteur le plus important est le pityrosporum ovale, levure commune à tous les cuirs chevelus mais dont les proportions augmentent très fortement dans tous les états pelliculaires, jusqu'à atteindre des proportions supérieures à soixante-quinze pour cent par rapport à la flore microbienne totale.

Le rôle du pityrosporum ovale est mal connu et on ne sait toujours pas s'il est un agent causal des états pelliculaires ou une conséquence. Il intervient indéniablement dans le processus car un traitement des états pelliculaires avec des antifongiques permet de revenir à un état normal.

Vraisemblablement, le Pityrosporum ovale, par les lipases qu'il produit, hydrolyse les triglycérides du sébum et de la peau, en acides gras libres.

Ces derniers sont plus sensibles que les triglycérides aux phénomènes de peroxydation.

Les peroxydes, formés par réactions radicalaires, sont des agents pro-inflammatoires.

L'inflammation qui en résulte provoquerait la prolifération cellulaire. De plus, le Pityrosporum est une levure lipophile et le sébum aurait donc un effet permissif sur sa croissance. On observe d'ailleurs les états pelliculaires dans les zones les plus riches en glandes sébacées. La séborrhée interviendrait donc indirectement dans les états pelliculaires.

Afin de compléter la revue des connaissances sur la question, on ajoute que le processus inflammatoire semble lié à tous les états pelliculaires. Il est plus manifeste dans les pityriasis dit stéatoïdes car on l'observe cliniquement.

Il est néanmoins réel dans les pityriasis simplex car même s'il n'est pas observable cliniquement, on décèle histologiquement une inflammation.

Sur la base de ces connaissances théoriques, la Demanderesse a pu réaliser des essais ciblés destinés à évaluer la qualité de compositions capillaires.

L'invention a pour but de fournir des compositions à base d'extraits de myrte possédant notamment des activités antipelliculaires, antiprurigineuses et antiséborrhéiques améliorées par rapport à celles qui existent déjà.

on y parvient, selon l'invention, en réalisant une composition capillaire comprenant, comme principe actif, un extrait de myrte comprenant des polyphénols en une quantité comprise entre 1 et 50 % en poids sec, par rapport à la matière sèche de l'extrait.

De préférence, l'extrait de myrte est présent en une proportion relative en poids, comprise entre 0,1 et 10 %, de préférence entre 0,5 et 8 % et plus préférentiellement encore entre 2 et 5 %.

Avantageusement, l'extrait de myrte comprend des polyphénols en une quantité comprise entre 10 et 40 % et de préférence entre 20 et 30 % en poids sec, par rapport à la matière sèche de l'extrait.

Selon un mode de réalisation avantageux de la composition selon l'invention, l'extrait de myrte comprend des flavonoïdes en une quantité comprise entre 0,1 et 5 %, de préférence entre 1 et 4 % et plus préférentiellement encore entre 2 et 3 % en poids sec, par rapport à la matière sèche de l'extrait.

Selon un mode de réalisation encore plus avantageux de la composition selon l'invention, l'extrait de myrte comprend des tanins en une quantité comprise entre 1 et 50 %, de préférence entre 5 et 40 % et plus préférentiellement encore entre 10 et 30 % en poids sec, par rapport à la matière sèche de l'extrait.

Avantageusement, l'extrait de myrte est un extrait de myrtus communis.

Le procédé de préparation d'une composition selon l'invention implique la fabrication d'un extrait de myrte à partir de feuilles et/ou de fruits de myrte, cette fabrication comprenant les étapes suivantes :
. un broyage des feuilles et/ou des fruits de myrte,
. une extraction des feuilles et/ou des fruits de myrte broyés par un solvant,
. une récupération de la solution d'extraction par filtration ou par essorage,
. une concentration de la solution récupérée par évaporation, pour obtenir l'extrait de myrte à mélanger, le cas échéant, à d'autres constituants, afin d'obtenir ladite composition.

De préférence, les feuilles et/ou les fruits de myrte sont des feuilles et/ou des fruits de myrte secs.

Plus préférentiellement, le rapport en poids des feuilles aux fruits de myrte est voisin de 90/10.

Dans l'étape d'extraction, le solvant utilisé est avantageusement un alcool en C₁ à C₄ ou l'acétone, ou un mélange eau-alcool ou un mélange acétone-eau, le rapport en poids des feuilles et/ou des fruits au solvant étant compris entre 1 et 20 et de préférence entre 1 et 4, la température d'extraction étant comprise entre la température ambiante et la température d'ébullition du solvant, et la durée d'extraction étant comprise entre 1 heure et 24 heures.

Selon un mode avantageux de mise en oeuvre du procédé selon l'invention, la concentration de la solution récupérée par évaporation est réalisée à pression réduite, à une température comprise entre 40 et 100°C jusqu'à l'obtention d'une poudre. On peut également, lors de l'opération de concentration, ajouter un solvant à haut point d'ébullition et plus particulièrement la glycérine, le propylène glycol, le butylène glycol ou le transcutol. L'extrait du myrte est alors sous forme liquide.

Les compositions selon l'invention peuvent être utilisées pour des traitements antipelliculaires, antiprurigineux, antiséborrhéiques, colorants ou antichutes des cheveux.

L'invention pourra être mieux comprise à l'aide des exemples non limitatifs qui suivent et qui constituent des modes de réalisation particuliers des compositions capillairesselon l'invention.

### 1. Exemples d'obtention de l'extrait

### Exemple 1 :

On met en oeuvre l'étape d'extraction conformément au procédé selon l'invention.

Pour cela, 100 kg de feuilles et fruits secs de myrte sont broyés puis extraits par 700 kg d'éthanol à 50 %. L'extraction est réalisée sous reflux pendant 1 heure et sous agitation. Les solutions sont récupérées après filtration, puis concentrées sous pression réduite à 60°C. Lors de la concentration, on rajoute 200 kg de propylène glycol, la concentration est menée jusqu'à l'obtention d'une solution pesant 220 kg. La teneur en matière sèche se situe entre 8 et 12 %, celle des polyphénols par rapport à la matière sèche entre 20 et 40 %, celle des flavonoïdes par rapport à la matière sèche, entre 1,5 et 3,5 %.

### Exemple 2 :

De même que pour l'exemple 1, on réalise un extrait de myrte, conformément au procédé selon l'invention. Néanmoins, on procède à partir de matière première différente de celle de l'exemple 1. On procède aussi au moyen d'un autre solvant d'extraction de manière à obtenir un extrait présentant des teneurs différentes en flavonoïdes et en polyphénols.

1 kg de feuilles sèches, broyées, est extrait par 10 kg de méthanol à froid pendant 12 heures. La solution méthanolique est concentrée sous vide à 60°C, puis séchée. L'extrait sec obtenu est broyé. La poudre titre entre 30 à 40 % en polyphénols et entre 3 et 4 % de flavonoïdes.

### Exemple 3 :

Dans cet exemple, on obtient encore un autre extrait.

10 kg de feuilles et fruits broyés sont extraits par un mélange d'eau et d'éthanol dans des proportions de 80 à 20 sous reflux et sous agitation pendant 3 heures. La solution obtenue est essorée puis filtrée. Sa teneur en polyphénols par rapport à la matière sèche se situe entre 5 et 10 %. Les flavonoïdes varient de 0,5 à 1,5 %.

### 2. Exemples de formulations

On réalise diverses compositions capillaires conformément à l'invention.

| Exemple 1 : SHAMPOOING | | |
|---|---|---|
| EXTRAIT DE MYRTE | 5 | g |
| ACIDE SALICYLIQUE | 1 | g |
| ALKYLETHER SULFATE DE SODIUM | 10 | g |
| COCAMIDOPROPYL BETAINE | 3 | g |
| DIETHANOLAMIDE D'ACIDES GRAS | 1 | g |
| ESSENCE DE GIROFLE | 0,05 | g |
| PARFUM | QS | |
| LAURAMINE OXYDE | 0,3 | g |
| MYRISTAMINE OXYDE | 0,5 | g |
| EAU PURIFIEE QSP | 100 | ml |

| Exemple 2 : SHAMPOOING | | |
|---|---|---|
| EXTRAIT DE MYRTE | 2 | g |
| DIETHANOLIMIDE UNDECYLENIQUE | 1 | g |
| ACIDE SALICYLIQUE | 1 | g |
| ALKYLETHER SULFATE DE SODIUM | 10 | g |
| ALKYL POLYGLUCOSIDE | 4 | g |
| COCAMIDOPROPYL BETAINE | 1,5 | g |
| DIETHANOLAMIDE D'ACIDES GRAS | 2 | g |
| DIMETHICONE COPOLYOL | 1 | g |
| LAURAMINE OXYDE | 1 | g |
| PARFUM | QS | |
| EAU PURIFIEE QSP | 100 | ml |

| Example 3 : SHAMPOOING | | |
|---|---|---|
| EXTRAIT DE MYRTE | 5 | g |
| ACIDE SALICYLIQUE | 0,5 | g |
| ACETAMIDE PEA | 0,5 | g |
| ALKYL POLYGLUCOSIDE | 7 | g |
| LIPOPROTEINES DE BLE | 8 | g |
| LAURYL AMIDE BETAINE | 1,5 | g |
| GLUCAMATE DOE/20 | 3 | g |
| LAURAMINE OXIDE | 1 | g |
| DIETHANOLAMIDE D'ACIDES GRAS | 1 | g |
| ESSENCE DE GIROFLE | 0,05 | g |
| EAU PURIFIEE QSP | 100 | ml |

| Exemple 4 : LOTION ANTIPELLICULAIRE | | |
|---|---|---|
| EXTRAIT DE MYRTE | 0,5 | g |
| PIROCTONOLAMINE | 0,02 | g |
| DIMETHICONE COPOLYOL | 0,20 | g |
| EXTRAIT DE CAPUCINE | 0,50 | g |
| ALCOOL 95 % | 53 | g |
| PARFUM | QS | |
| EAU PURIFIEE QSP | 100 | ml |

| Exemple 5 : BAUME RINCE ANTIPELLICULAIRE | | |
|---|---|---|
| EXTRAIT DE MYRTE | 5 | g |
| ACIDE SALICYLIQUE | 0,5 | g |
| STEARAMINE OXYDE | 3 | g |
| LAURAMINE OXYDE | 1 | g |
| ACIDE CHLORHYDRIQUE ET CHLORURE DE POTASSIUM | QS | |
| LACTAMIDE MEA | 0,50 | g |
| PARFUM | QS | |
| EAU PURIFIEE QSP | 100 | ml |

| Exemple 6 : BAUME RINCE APAISANT | | |
|---|---|---|
| EXTRAIT DE MYRTE | 5 | g |
| EXTRAIT DE CALENDULA | 1 | g |
| STEARAMINE OXYDE | 3 | g |
| LAURAMINE OXYDE | 1 | g |
| ACIDE CHLORHYDRIQUE CHLORURE DE POTASSIUM | QS | |
| ACETAMIDE MEA | 0,50 | g |
| PARFUM | QS | |
| EAU PURIFIEE QSP | 100 | ml |

| Exemple 7 : LOTION STIMULANTE ANTICHUTE | | |
|---|---|---|
| EXTRAIT DE MYRTE | 5 | g |
| EXTRAIT D'ORTIE DIOIQUE | 10 | g |
| EXTRAIT DE CENTELLA ASIATICA | 0,30 | g |
| EXTRAIT D'ECHINACEA | 10 | g |
| NICOTINAMIDE | 0,10 | g |
| VITAMINE B6 | 0,20 | g |
| PANTHENOL | 0,30 | g |
| PCA ZINC | 0,20 | g |
| HUILE ESSENTIELLE DE ROMARIN | 0,05 | g |
| HUILE ESSENTIELLE D'ORANGE | 0,05 | g |
| ETHOXYDIGLYCOL | 10 | g |
| ALCOOL 60 % VOL. QSP | 100 | g |

| Exemple 8 : SHAMPOOING REFLET AUX EXTRAITS NATURELS | | |
|---|---|---|
| EXTRAIT DE HENNE | 5 | % |
| EXTRAIT DE SANTAL | 1,5 | % |
| EXTRAIT DE NOYER | 3 | % |
| EXTRAIT DE MYRTE | 5 | % |
| ALKYL ETHER SULFATE DE SODIUM | 10 | g |
| COCAMIDOPROPYL BETAINE | 3 | g |
| ALKYL POLYGLUCOSIDE | 2 | g |
| BASE NACRANTE | 6 | g |
| ALKANOLAMIDE DE COPRAH | 2 | g |
| LAURAMINE OXYDE | 2 | g |
| EAU PURIFIEE QSP | 100 | ml |

(les pourcentages indiqués sont des pourcentages pondéraux).

### Evaluation des activités des compositions selon l'invention

On évalue diverses activités des extraits de myrte selon l'invention.

On évalue notamment les activités antipelliculaires, antiséborrhéiques, antiprurigineuses, colorantes et antichutes.

### 1. Activité antipelliculaire

Pour évaluer l'activité antipelliculaire, on se réfère aux activités anti-inflammatoires, antiradicalaires et antifongiques.
a) Activité anti-inflammatoire
   L'activité anti-inflammatoire des extraits de myrte a été évaluée sur le kératinocyte. Ce dernier constitue la cellule la plus représentée au niveau de l'épiderme. En réponse à de nombreux stimuli extra-cellulaires présents dans un environnement, il libère divers médiateurs biologiquement actifs, notamment les prostaglandines et les leucotriènes qui jouent un rôle important dans l'initiation et la modulation des réactions inflammatoires cutanées et qui interviennent également dans la régulation de la réponse immune.
   Le kératinocyte se révèle être un bon modèle d'étude en pharmacologie cutanée ; ce modèle cellulaire permet de déterminer in vitro les capacités de diverses molécules à moduler la production de ces médiateurs issus du métabolisme de l'acide arachidonique.
   Dans cette étude, on a visé en particulier une prostaglandine, la PG6KF1α qui est un des métabolites majeurs produits par le kératinocyte stimulé, et représentatif de la modulation de la production des métabolites de l'acide arachidonique issus de la voie de la cyclo-oxygénase.
   L'effet d'un extrait de myrte dosé à 30 % en polyphénols et 2,5 % en flavonoïdes sur la production de PG6KF1α induite chez le kératinocyte a été étudié. Ce dernier est induit par deux agonistes qui stimulent en synergie la cascade de l'acide arachidonique : ionophore calcique A23187, qui induit lui-même la mobilisation de calcium et un ester de phorbol, le TPA qui active quant à lui la protéine kinase C.
   Cette étude de la modulation a été effectuée in vitro, après exposition à l'extrait de myrte, de la libération de la prostaglandine 6KF1α par les kératinocytes stimulés. On observe que l'extrait de myrte inhibe de façon significative, aux concentrations 10 et 50 µg/ml, la production de prostaglandine 6KF1α induite par l'ester de phorbol TPA et le ionophore calcique A23187. Cette inhibition se révèle être proportionnelle à la concentration de l'extrait de myrte. On note par exemple que 29 % d'inhibition est obtenu pour la concentration 10 µg/ml et que 57 % d'inhibition est obtenu pour la concentration 50 µg/ml.
   Cette inhibition par l'extrait de myrte de la production de PG6KF1α, médiateur majeur de la réponse inflammatoire prouve l'activité anti-inflammatoire de cet extrait.
b) Activité antiradicalaire
   Il est bien connu que la réaction radicalaire se divise en trois étapes successives, à savoir l'initiation, la propagation et la disparition des radicaux libres. Dans le détail, on précise que l'initiation est due en l'occurrence à l'anion superoxyde 0²-, qui apparaît sous l'effet de facteurs tels que le rayonnement UV ou le stress.
   La propagation est le fait des radicaux hydroxyles OH-. Lors de la disparition des radicaux libres, les piégeurs de radicaux libres agissent sur l'anion superoxyde mais également sur les radicaux hydroxyles.
   L'activité antiradicalaire a été évaluée sur les deux premières étapes.
   L'activité sur l'anion superoxyde est réalisée par test in vitro. L'anion superoxyde est généré par photo-oxydation radicalaire, par sensibilisation de la riboflavine au rayonnement visible.
   L'indicateur coloré utilisé est le nitrobleu de tétrazolium, électrophile qui est réduit par l'anion superoxyde généré en diformazan.
   On évalue l'activité antiradicalaire de l'extrait de myrte préparé conformément à l'invention. On observe que cet extrait inhibe 50 % de l'activité réductrice de l'anion superoxyde sur le NBT. Par ailleurs, la CI50 est 0,09 mg/ml.
   L'activité sur la propagation radicalaire, donc sur les radicaux hydroxyles, est évaluée sur le diphényl picryl hydrazyl hydrate (DPPH), radical libre stable. La CI50 d'un extrait de myrte titrant à 30 % en polyphénols totaux et 2,5 % en flavonoïdes est 0,005 mg/ml. La CI50 de la vitamine E, molécule de référence dans ce test est de 0,006 mg/ml. L'extrait de myrte a donc une activité antiradicalaire comparable à la vitamine E.
   On montre ainsi l'efficacité de l'extrait de myrte en tant que composition antiradicalaire.
c) Activité antifongique
   L'activité antifongique des extraits de myrte a été recherchée sur la levure mise en évidence in vivo lors d'états pelliculaires.
   La culture de Pityrosporum ovale a lieu sur milieu Dixon à 32°C.
   Il s'agit d'une souche sauvage recueillie à l'Hôpital de Rangueil, Toulouse (France) dans le Service de Parasitologie.
   L'étude des extraits a lieu par recherche de la concentration minimale inhibitrice de la croissance de la souche en milieu Dixon liquide par rapport à un témoin souche, celui-ci étant sans extrait et un témoin de stérilité du milieu, celui-ci étant sans souche, ni extrait. La culture du germe se fait en microméthode, pendant 72 h à 32°C.
   0,04 % d'un extrait de myrte dosé à 30 % en polyphénols et 2,5 % en flavonoïdes, inhibent la croissance de la levure après 72 h de contact. Une concentration de 0,12 % est fongicide sur la levure testée, après repiquage des dilutions sur Dixon solide.
   On démontre ainsi l'activité antifongique de l'extrait de myrte.

### 2. Activité antiséborrhéique

Cette activité est classique pour des extraits riches en tanins. En effet, ces derniers présentent une forte activité astringente. Cette activité va donc normalement provoquer une contraction des tissus et, par-là même, une diminution de sécrétions sébacées par simple phénomène physique puisque le sébum ne pourra s'écouler facilement.

L'extrait de Myrtus, par sa richesse en tanins qui se trouve à la hauteur de 5 à 40 %, présente donc forcément une activité antiséborrhéique.

Cette activité a d'ailleurs pu être mise en évidence par la Demanderesse de la manière suivante. Elle a évalué cette activité sur dix volontaires masculins. Dans un premier temps, vingt cheveux sont prélevés sur chaque volontaire. Ce prélèvement permettra de doser la quantité de sébum présente initialement sur les cheveux.

Les volontaires ont dans un second temps une demi- tête lavée avec le shampooing antiséborrhéique décrit dans l'exemple 3, l'autre demi-tête est lavée avec le même shampooing sans extrait de myrte. Cette opération est répétée pendant cinq jours, une fois par jour. A la fin du traitement, vingt cheveux sont prélevés sur chaque demi-tête. Tous les cheveux sont extraits de la même façon avec un solvant apolaire et on dose par chromatographie gazeuse le squalène présent dans les solutions. Le squalène est le composant principal du sébum. Ce dosage permet donc d'évaluer l'activité antiséborrhéique d'une composition cosmétique ou d'un actif.

Ainsi, chez tous les volontaires, on observe une diminution de la quantité de squalène donc de sébum sur les cheveux traités avec les deux shampooings mais de façon beaucoup plus prononcée sur les cheveux traités avec le shampooing contenant l'extrait de myrte dosé à 30 % en polyphénols et 2,5 % en flavonoïdes.

L'activité antiséborrhéique de l'extrait de myrte est ainsi démontrée.

### 3. Activité antiprurigineuse

La recherche de l'activité antiprurigineuse a été effectuée sur dix volontaires sains.

La sensation de prurit est déclenchée par un stimulus thermique et on note le délai d'apparition de la sensation de prurit ainsi que sa durée.

Les informations concernant le prurit sont évaluées par le volontaire oralement.

L'extrait de myrte dosé à 30 % en polyphénols et 2,5 % en flavonoïdes selon l'exemple 1 retarde le délai d'apparition de la sensation de prurit chez 85 % des volontaires contre le placebo - et la durée de la sensation de prurit a diminué chez 90 % des volontaires par rapport au placebo.

L'extrait de myrte selon l'invention provoque donc une diminution sensible de la perception du prurit, en retardant son apparition et en écourtant sa durée.

### 4. Activité colorante

L'effet renforçateur de l'extrait de myrte sur des reflets de coloration a été évalué.

Pour ce faire, le test a été mené sur dix volontaires féminins en cabine. Chaque volontaire est traité sur une demi-tête avec une solution contenant 5 % d'extrait de henné, 1,5 % d'extrait de santal, 3 % d'extrait de noyer et 5 % d'extrait de myrte.

L'autre demi-tête est traitée avec la même solution sans extrait de myrte. Après traitement, les deux demi-têtes sont rincées et on observe comparativement les deux colorations.

Dans tous les cas, les reflets obtenus sont plus soutenus pour les demi-têtes traitées avec la solution contenant l'extrait de myrte que celles traitées avec la solution sans extrait de myrte.

Par ce test, l'effet renforçateur de coloration de l'extrait de myrte est démontré.

### 5. Activité antichute

L'extrait de myrte est testé à 10 % dans une solution hydroalcoolique à 35 % en volume. Cette solution renferme en outre un agent cationique permettant d'avoir un bon effet mouillant, donc une meilleure pénétration.

Pour évaluer l'activité antichute de l'extrait, on sélectionne vingt sujets masculins présentant un début d'alopécie d'un niveau II ou III.

Avant le traitement proprement dit, tous les sujets utilisent pendant trois semaines un shampooing neutre sans autre produit. Cette précaution permet de ramener les sujets au même niveau de départ.

La solution de myrte est appliquée sur le cuir chevelu trois fois par semaine à raison de 1 ml à 1,5 ml par application, cette dernière étant accompagnée d'un massage.

La durée du traitement est ainsi de trois mois.

Afin de quantifier l'action de l'extrait de myrte sur la chute des cheveux, on mesure, d'une part, le diamètre des cheveux en phase anagène, c'est-à-dire en cours de croissance.

D'autre part, on mesure la densité pilaire effectuée sur une zone parfaitement repérée et délimitée.

On effectue ces mesures chez chaque sujet avant le début du traitement puis après trois mois de traitement.

La comparaison des valeurs obtenues avant et après le traitement révèle, de façon significative, l'intérêt de l'utilisation de l'extrait de myrte dans le soin de l'alopécie.

## Revendications

1. Composition capillaire, **caractérisée par le fait qu'**elle comprend, comme principe actif, un extrait de myrte comprenant des polyphénols en une quantité comprise entre 1 et 50 % en poids sec, par rapport à la matière sèche de l'extrait.

2. Composition selon la revendication 1, **caractérisée par le fait que** l'extrait de myrte est présent en une proportion relative en poids, comprise entre 0,1 et 10%, de préférence entre 0,5 et 8% et plus préférentiellement encore entre 2 et 5%.

3. Composition selon l'une quelconque des revendications précédentes, **caractérisée par le fait que** l'extrait de myrte comprend des polyphénols en une quantité comprise entre 10 et 40% et de préférence, entre 20 et 30% en poids sec, par rapport à la matière sèche de l'extrait.

4. Composition selon l'une quelconque des revendications précédentes, **caractérisée par le fait que** l'extrait de myrte comprend des flavonoïdes en une quantité comprise entre 0,1 et 5%, de préférence entre 1 et 4% et plus préférentiellement encore entre 2 et 3% en poids sec, par rapport à la matière sèche de l'extrait.

5. Composition selon l'une quelconque des revendications précédentes, **caractérisée par le fait que** l'extrait de myrte comprend des tanins en une quantité comprise entre 1 et 50%, de préférence entre 5 et 40% et plus préférentiellement encore entre 10 et 30% en poids sec, par rapport à la matière sèche de l'extrait.

6. Composition selon l'une quelconque des revendications précédentes, **caractérisée par le fait que** l'extrait de myrte est un extrait de Myrtus communis.

7. Procédé de préparation d'une composition selon Tune quelconque des revendications précédentes, **caractérisé par le fait qu'**il implique la fabrication d'un extrait de myrte à partir de feuilles et/ou de fruits de myrte, cette fabrication comprenant les étapes suivantes :
. un broyage des feuilles et/ou des fruits de myrte,
. une extraction des feuilles et/ou des fruits de myrte broyés par un solvant,
. une récupération de la solution d'extraction par filtration ou par essorage,
. une concentration de la solution récupérée par évaporation, pour obtenir l'extrait de myrte à mélanger, le cas échéant, à d'autres constituants, afin d'obtenir ladite composition.

8. Procédé selon la revendication 7, **caractérisé par le fait que** les feuilles et/ou les fruits de myrte sont des feuilles et/ou des fruits de myrte secs.

9. Procédé selon Tune quelconque des revendications 7 ou 8, **caractérisé par le fait que** le rapport en poids des feuilles aux fruits de myrte est voisin de 90/10.

10. Procédé selon Tune quelconque des revendications 7 à 9, **caractérisé par le fait que** dans l'étape d'extraction, le solvant utilisé est un alcool en C₁ à C₄ ou l'acétone, ou un mélange eau-alcool ou un mélange acétone-eau, le rapport en poids des feuilles et/ou des fruits au solvant étant compris entre 1 et 20 et de préférence entre 1 et 4, la température d'extraction étant comprise entre la température ambiante et la température d'ébullition du solvant, et la durée d'extraction étant comprise entre 1 heure et 24 heures.

11. Procédé selon Tune quelconque des revendications 7 à 10, **caractérisé par le fait que** la concentration de la solution récupérée par évaporation est réalisée à pression réduite, à une température comprise entre 40 et 100°C jusqu'à l'obtention d'une poudre.

12. Procédé selon l'une quelconque des revendications 7 à 11, **caractérisé par le fait que** lors de l'étape de concentration de la solution, on ajoute un solvant à haut point d'ébullition, notamment un solvant choisi dans le groupe comprenant la glycérine, le propylène glycol, le butylène glycol ou le transcutol, afin de recueillir un extrait liquide.

13. Utilisation d'une composition capillaire selon l'une quelconque des revendications 1 à 6, ou résultant de la mise en oeuvre du procédé selon Tune des revendications 7 à 12, pour la fabrication d'un médicament destiné aux traitements antipelliculaires, antiprurigineux, antiséborrhéiques, colorants ou antichutes des cheveux.

14. Composition capillaire sous la forme d'une lotion antipelliculaire contenant 0,5 g d'extrait de myrte, 0,02 g de piroctonolamine, 0,20 g de diméthicone copolyol, 0,50 g d'extrait de capucine, 53 g d'alcool à 95 % et une quantité suffisante d'eau purifiée pour obtenir un volume de 100 ml.

## Patentansprüche

1. Haar-Zusammensetzung, **dadurch gekennzeichnet, dass** sie als Wirkstoff einen Myrtenextrakt umfasst, der Polyphenole in einer Menge von 1 bis 50 Trockengewichts-%, bezogen auf das trockene Material des Extrakts, umfasst.

2. Zusammensetzung nach Anspruch 1, **dadurch gekennzeichnet, dass** der Myrtenextrakt in einem relativen Gewichtsanteil von 0,1 bis 10%, vorzugsweise von 0,5 bis 8% und noch bevorzugter von 2 bis 5% anwesend ist.

3. Zusammensetzung nach irgendeinem der vorangehenden Ansprüche, **dadurch gekennzeichnet, dass** der Myrtenextrakt Polyphenole in einer Menge von 10 bis 40 und vorzugsweise von 20 bis 30 Trockengewichts-%, bezogen auf das trockene Material des Extrakts, umfasst.

4. Zusammensetzung nach irgendeinem der vorangehenden Ansprüche, **dadurch gekennzeichnet, dass** der Myrtenextrakt Flavonoide in einer Menge von 0,1 bis 5, vorzugsweise von 1 bis 4 und noch bevorzugter von 2 bis 3 Trockengewichts-%, bezogen auf das trockene Material des Extrakts, umfasst.

5. Zusammensetzung nach irgendeinem der vorangehenden Ansprüche, **dadurch gekennzeichnet, dass** der Myrtenextrakt pflanzliche Gerbstoffe in einer Menge von 1 bis 50, vorzugsweise von 5 bis 40 und noch bevorzugter von 10 bis 30 Trockengewichts-%, bezogen auf das trockene Material des Extrakts, umfasst.

6. Zusammensetzung nach irgendeinem der vorangehenden Ansprüche, **dadurch gekennzeichnet, dass** der Myrtenextrakt ein Extrakt von Myrtus communis ist.

7. Verfahren zur Herstellung einer Zusammensetzung nach irgendeinem der vorangehenden Ansprüche, **dadurch gekennzeichnet, dass** es die Herstellung eines Myrtenextrakts ausgehend von Blättern und/oder Früchten von Myrte beinhaltet, wobei diese Herstellung die folgenden Schritte umfasst:
- Zermahlen der Myrtenblätter und/oder -früchte,
- Extraktion der zermahlenen Myrtenblätter und/oder -früchte durch ein Lösungsmittel,
- Gewinnung der Extraktionslösung durch Filtration oder durch Auswringen,
- Konzentration der gewonnenen Lösung durch Eindampfen, um den Myrtenextrakt zu erhalten, der gegebenenfalls mit anderen Bestandteilen zu mischen ist, um die Zusammensetzung zu erhalten.

8. Verfahren nach Anspruch 7, **dadurch gekennzeichnet, dass** die Myrtenblätter und/oder -früchte trockene Myrtenblätter und/oder -früchte sind.

9. Verfahren nach irgendeinem der Ansprüche 7 oder 8, **dadurch gekennzeichnet, dass** das Gewichtsverhältnis von Myrtenblättern zu -früchten etwa 90/10 beträgt.

10. Verfahren nach irgendeinem der Ansprüche 7 bis 9, **dadurch gekennzeichnet, dass** in dem Extraktionsschritt das verwendete Lösungsmittel ein C₁- bis C₄-Alkohol oder Aceton oder eine Mischung Wasser-Alkohol oder eine Mischung Aceton-Wasser ist, das Gewichtsverhältnis der Blätter und/oder der Früchte zum Lösungsmittel zwischen 1 und 20 und vorzugsweise zwischen 1 und 4 eingeschlossen ist, die Extraktionstemperatur zwischen Umgebungstemperatur und dem Siedepunkt des Lösungsmittels eingeschlossen ist und die Extraktionsdauer zwischen 1 Stunde und 24 Stunden eingeschlossen ist.

11. Verfahren nach irgendeinem der Ansprüche 7 bis 10, **dadurch gekennzeichnet, dass** die Konzentration der gewonnenen Lösung durch Eindampfen bei verringertem Druck bei einer Temperatur von 40 bis 100°C bis zum Erhalt eines Pulvers durchgeführt wird.

12. Verfahren nach irgendeinem der Ansprüche 7 bis 11, **dadurch gekennzeichnet, dass** man während des Schrittes der Konzentration der Lösung ein Lösungsmittel mit hohem Siedepunkt zusetzt, insbesondere ein Lösungsmittel, das ausgewählt ist aus der Gruppe, die Glycerin, Propylenglycol, Butylenglycol oder Transcutol umfasst, um einen flüssigen Extrakt zu erhalten.

13. Verwendung einer Haar-Zusammensetzung nach irgendeinem der Ansprüche 1 bis 6 oder resultierend aus der Durchführung des Verfahrens nach einem der Ansprüche 7 bis 12 für die Herstellung eines Medikaments, das zu Antischuppen-, Antijuckreiz-, Antiseborrhö-, färbenden oder Antihaarausfall-Behandlungen bestimmt ist.

14. Haar-Zusammensetzung in Form einer Antischuppen-Lotion, die 0,5 g Myrtenextrakt, 0,02 g Piroctonolamin, 0,20 g Dimethiconcopolyol, 0,50 g Kapuzinerkresseextrakt, 53 g 95%-igen Alkohol und eine ausreichende Menge an gereinigtem Wasser umfasst, um ein Volumen von 100 ml zu erhalten.

## Claims

1. Hair composition, **characterized in that** it comprises, as active principle, an extract of myrtle comprising polyphenols in an amount of between 1% and 50% by dry weight, relative to the dry matter of the extract.

2. Composition according to Claim 1, **characterized in that** the extract of myrtle is present in a relative proportion by weight of between 0.1% and 10%, preferably between 0.5% and 8% and even more preferably between 2% and 5%.

3. Composition according to either of the preceding claims, **characterized in that** the extract of myrtle comprises polyphenols in an amount of between 10% and 40% and preferably between 20% and 30% by dry weight, relative to the dry matter of the extract.

4. Composition according to any one of the preceding claims, **characterized in that** the extract of myrtle comprises flavonoids in an amount of between 0.1% and 5%, preferably between 1% and 4% and even more preferably between 2% and 3% by dry weight, relative to the dry matter of the extract.

5. Composition according to any one of the preceding claims, **characterized in that** the extract of myrtle comprises tannins in an amount of between 1% and 50%, preferably between 5% and 40% and even more preferably between 10% and 30% by dry weight, relative to the dry matter of the extract.

6. Composition according to any one of the preceding claims, **characterized in that** the extract of myrtle is an extract of Myrtus communis.

7. Process for preparing a composition according to any one of the preceding claims, **characterized in that** it involves the manufacture of an extract of myrtle from myrtle leaves and/or fruit, this manufacture comprising the following steps:
• grinding the myrtle leaves and/or fruit,
• extracting the ground myrtle leaves and/or fruit with a solvent,
• recovering the extraction solution by filtration or draining,
• concentrating the recovered solution by evaporation, to obtain the extract of myrtle to be mixed, where appropriate, with other constituents, in order to obtain the said composition.

8. Process according to Claim 7, **characterized in that** the myrtle leaves and/or fruit are dry myrtle leaves and/or fruit.

9. Process according to either of Claims 7 and 8, **characterized in that** the weight ratio of the myrtle leaves to fruit is in the region of 90/10.

10. Process according to any one of Claims 7 to 9, **characterized in that**, in the extraction step, the solvent used is a C₁ to C₄ alcohol or acetone, or a water-alcohol mixture or an acetone-water mixture, the weight ratio of the leaves and/or fruit to the solvent being between 1 and 20 and preferably between 1 and 4, the extraction temperature being between room temperature and the boiling point of the solvent, and the extraction time being between 1 hour and 24 hours.

11. Process according to any one of Claims 7 to 10, **characterized in that** the concentration of the recovered solution by evaporation is performed under reduced pressure, at a temperature of between 40 and 100°C, until a powder is obtained.

12. Process according to any one of Claims 7 to 11, **characterized in that** during the step of concentrating the solution, a high-boiling solvent is added, especially a solvent chosen from the group comprising glycerol, propylene glycol, butylene glycol and transcutol, in order to collect a liquid extract.

13. Use of a hair composition according to any one of Claims 1 to 6, or resulting from carrying out the process according to one of Claims 7 to 12, for the manufacture of a medicinal product for anti-dandruff, anti-pruriginous, anti-seborrhoeic or hair-dyeing treatments or treatments for preventing hair loss.

14. Hair composition in the form of an anti-dandruff lotion containing 0.5 g of extract of myrtle, 0.02 g of piroctonolamine, 0.20 g of dimethicone copolyol, 0.50 g of extract of nasturtium, 53 g of 95% alcohol and a sufficient amount of purified water to obtain a volume of 100 ml.
